(19) Europäisches Patentamt
European Patent Office
Office européen des brevets

(11) EP 0 894 489 A1

(12) **EUROPEAN PATENT APPLICATION**

(43) Date of publication:
**03.02.1999 Bulletin 1999/05**

(51) Int Cl.$^6$: **A61K 7/00**

(21) Application number: **98305161.6**

(22) Date of filing: **30.06.1998**

(84) Designated Contracting States:
**AT BE CH CY DE DK ES FI FR GB GR IE IT LI LU MC NL PT SE**
Designated Extension States:
**AL LT LV MK RO SI**

(30) Priority: **30.06.1997 US 884831**

(71) Applicant: **Calgon Corporation**
**Pittsburgh, Pennsylvania 15205 (US)**

(72) Inventors:
- **Blanco, Beatriz**
  **Pittsburgh, Pennsylvania 15243 (US)**
- **Matz, Gary F.**
  **Carnegie, Pennsylvania 15106 (US)**

(74) Representative: **McCormack, Derek James**
**ECC International Ltd,**
**Patents Department,**
**c/o John Keay House**
**St Austell, Cornwall PL25 4DJ (GB)**

(54) **Cleansing compositions comprising ampholyte terpolymers and methods of using the same**

(57) A cleansing composition comprising a cosmetically acceptable medium containing from 0.05 per cent by weight to 10 per cent by weight of one or more ampholyte terpolymers having a weight average molecular weight of from 10,000 to 10,000,000 comprising:

(a) a non-ionic monomer forming from 1 per cent to 95 per cent by weight of the terpolymer;
(b) a cationic monomer forming from 5 per cent to 80 per cent by weight of the terpolymer; and
(c) an anionic monomer forming from 1 per cent to 75 per cent of the terpolymer;

and wherein the cosmetically acceptable medium comprises a medium suitable for providing a hydrophilic cleansing product for use in direct contact with the skin of a user but not primarily for hair care, the medium containing a surfactant. The composition may be used in skin cleansing compositions to cleanse the skin of a user.



Printed by Jouve, 75001 PARIS (FR)

## Description

## BACKGROUND OF THE INVENTION

### Field of the Invention

The present invention relates to compositions and methods for reducing dermal and ocular irritation in various cleansing products. More specifically, the present invention relates to the use of ampholyte terpolymers having superior dermal and ocular irritation reduction properties when used in various cleansing formulations.

### Background Information

The surface properties of human skin and nails are, of course, of basic interest in cosmetic science, and there has thus been a long-standing desire to discover ingredients that will beneficially affect the topical condition of these keratinous substrates. Skin care and conditioning products are desired that will function to improve such properties as retention of skin moisture, softening of the skin, attraction of air moisture, retardation of skin water loss, feel and reduction of skin irritations caused by contact with detergents, soaps and the like.

The cleansing of skin with preparations containing surfactants has become an area of great interest. Cleansers such as shower gels, soap bars, bath oil bars, liquid hand soaps and vaginal douches are composed of a mixture of natural and synthetic surfactants. Many people wash and scrub their skin with various surfactant-containing products several times a day. In addition, the skin is in contact with other cleansing products, such as dish detergents and laundry detergents, which contain surfactant systems. Ideal cleansing products should clean the skin gently, causing little or no irritation to the skin or eyes, without defatting or overdrying the skin or otherwise leaving it taut after frequent routine use. Soap and surfactant residue which is retained on the skin after washing has been shown to cause skin roughness, tightness and drying. Most skin cleansers are irritating to the skin and eyes. Certain synthetic and natural surfactants are particularly mild and can be used to formulate a mild skin cleanser. A major drawback of most mild synthetic surfactant systems when formulated for skin cleansing is poor lather performance. High sudsing anionic surfactants are poor in clinical mildness.

Terpolymers of acrylamide (AM), dimethyldiallylammonium chloride (DMDAAC), and acrylic acid (AA) are disclosed in US Patent Nos. 4,455,240; 4,460,677; 4,484,631; 4,533,708; and 5,296,218. None of these patents suggest that such terpolymers might be used as agents for reducing dermal or ocular irritation in detergent or cleansing formulations.

US Patent No 4,110,263 relates to a mild cleansing composition comprising at least one detergent and at least one alkyleneoxylated bisquaternary ammonium compound. The addition of the ammonium compound to anionic and/or amphoteric detergents is reported to reduce the irritant properties of the detergents. US Patent No 4,181,634, which is a continuation-in-part of 4,110,263, also discloses a mild cleansing composition comprising an anionic and/or amphoteric detergent and an alkyleneoxylated bisquaternary ammonium compound which reduces the irritant properties of detergents. A specific cosmetic cleaning composition utilizing an amphoteric detergent is disclosed.

US Patent No 4,554,098 relates to a liquid detergent composition having good foaming and grease emulsification properties as well as reduced skin irritation effects. These compositions consist essentially of a water soluble C10-C16 alkylether ethylenoxy sulfate salt containing an average of 5 to 12 moles of ethylene oxide in the molecule, a supplementary, water soluble, non soap, anionic detergent having in its molecular structure a C8-C22 alkyl alkenyl or acyl group, and a sulfonate, sulfate or carboxylate group and a zwitterionic detergent.

US Patent No 4,603,005 relates to the use of alpha olefin/maleic anhydride terpolymers in conjunction with anionic surfactants in hair, skin and other cleaning compositions. The terpolymers in these compositions reduce the irritation potential of anionic surfactants in the compositions.

US Patent No 4,673,525 relates to an ultra-mild skin cleansing toilet bar composition comprising mild synthetic surfactants, a moisturizer, soap and a polymeric skin mildness aid. These mildness aids include cationic, anionic, nonionic and amphoteric polymers. The patent does not disclose the use of the amphoteric terpolymers disclosed herein.

US Patent No 4,764,365 relates to improved personal skin care products containing a DMDAAC/AA type polymer. These polymers provide exceptional feel to the skin when incorporated into personal skin care products.

US Patent No 4,772,462 relates to improved hair products containing DMDAAC/AA type polymers. These polymers provide exceptional conditioning properties to hair care products.

US Patent No 5,275,809 discloses ampholyte terpolymer conditioning additives for hair care products comprising AM, DMDAAC and the anionic monomer 2-acrylamido-2-methylpropane sulfonic acid (AMPSA) with optionally up to 50 percent of said anionic monomer AMPSA being replaced with the anionic monomer AA.

US Patent No 5,338,541 discloses the use of a terpolymer comprised of AM, DMDAAC, and second cationic

monomer. These terpolymers provide superior conditioning properties in hair, skin and nail care products.

US Patent No 5,480,633 relates to a skin cleanser and conditioner composition that removes soap and surfactant residues from the skin thereby preventing the irritation caused by the deposition of soaps and surfactants thereon. These compositions consist of water, low levels of a nonionic surfactant as the sole surfactant, low levels of an organic acid having a pKa from 4.5 to 6.5 and a pH of 4.5 to 6.5.

US Patent No 5,609,862 discloses ampholyte terpolymers having a nonionic monomer, a cationic monomer and an anionic monomer having an overall net charge between -5.0 and +5.0. The ampholyte terpolymers are added to hair care product formulations in amounts ranging from 0.1 to 10 percent by weight to provide superior conditioning properties to the products.

Japanese Patent JP-08283785 discloses cosmetic cleaners for hair and skin comprising anionic surfactants, amine oxides and cationic acrylate copolymers. The patent does not disclose use of an amphoteric terpolymer for the reduction of dermal or ocular irritation in a cleansing product. Japanese Patent JP-08283127 discloses hair cleanser compositions having amine oxides, amphoteric polymers and cationic surfactants. Again, this does not teach the product or methods of the present invention.

None of the above patents disclose the use of ampholyte polymers as an agent to reduce dermal and/or ocular irritation in cleansing products. Accordingly, there remains a need for such products, which provide improved qualities to the consumer.

**SUMMARY OF THE INVENTION**

The present invention meets the above described need by providing use of an ampholyte terpolymer in a cleansing formulation, whereby a milder cleansing product having reduced dermal and ocular irritation results. Various formulations comprising these terpolymers may be provided.

According to the present invention in a first aspect there is provided a cleansing composition comprising a cosmetically acceptable medium containing from 0.05 per cent by weight to 10 per cent by weight of one or more ampholyte terpolymers having a weight average molecular weight of from 10,000 to 10,000,000 comprising:

(a) a non-ionic monomer forming from 1 per cent to 95 per cent by weight of the terpolymer;
(b) a cationic monomer forming from 5 per cent to 80 per cent by weight of the terpolymer and (c) an anionic monomer forming from 1 per cent to 75 per cent of the terpolymer;

and wherein the cosmetically acceptable medium comprises a medium suitable for providing a hydrophilic cleansing product for use in direct contact with the skin of a user but not primarily for hair care, the medium containing a surfactant.

According to the present invention in another aspect there is provided a method of use of such a composition which comprises cleaning the skin by applying the composition to the skin in or as a cosmetically acceptable medium. The ampholyte terpolymers employed in the compositions and method of use of the present invention represent a significant advance in the state of the art of cleansing products and afford properties which are a surprising improvement over those possessed by the cleansing products of the prior art.

By the present invention mild soap products having reduced dermal and ocular irritation properties may be provided.

Also, by the invention it is possible to provide cleansing formulations having reduced irritation properties.

Further, the present invention provides mild cleansing products containing the said ampholyte terpolymer in conjunction with a surfactant system.

The present invention also provides a method of use of the said composition wherein the said composition has reduced dermal and ocular irritation properties.

**Components of the Ampholyte Terpolymers**

Turning to each of the components of the ampholyte terpolymer employed in the compositions according to the present invention, the nonionic monomer may comprise from 1 to 3 members independently selected from the group consisting of the following monomers and derivatives thereof:

| | |
|---|---|
| acrylamide (AM) | vinylacetate (VA) |
| N-alkylacrylamide (NAAM) | vinyl alcohol (VOH) |
| N-vinylpyrrolidinone (VP) | acrylate esters |
| methacrylamide (MAM) | allyl alcohol (AAlc) |
| methyl acrylate (MA) and methacrylate esters | methyl methacrylate (MMA) |

Derivatives of the above monomers are well known and are within the scope of component (a) of the terpolymer. For example, N-alkylacrylamide and methacrylamide may be regarded as derivatives of acrylamide, and these together with other known derivatives of acrylamide suitable for use in the ampholyte terpolymers of the present invention may be represented by the following general formula:

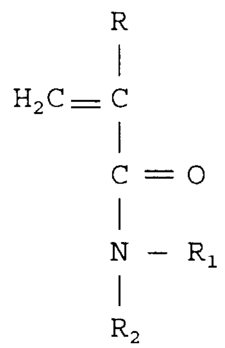

(1)

where R is H or $CH_3$; and $R_1$ and $R_2$ are independently H, $C_{1-4}$ alkyl, $CH_2OCH_3$, $CH_2OCH_2CH(CH_3)_2$, $(CH_2CH_2O\text{--})_x$--H, where x=1 to 50, or phenyl, or together are $C_{3-6}$cycloalkyl.

The preferred acrylamide monomer is the simplest, ie, that where R, $R_1$ and $R_2$ are all H. However, the other acrylamide derivatives within the scope of the formula set out above are also contemplated to be a part of the present invention, since they are well known in the art of water soluble polymers, where polyacrylamide and copolymers using acrylamide monomer are well known.

The nonionic acrylamide monomer portion of the ampholyte terpolymers employed in compositions embodying the present invention is present in an amount of from 1 to 95 weight percent of the total terpolymer. Preferably, this amount is from 5 to 80 weight percent, and more preferably this amount is from 10 to 50 weight percent and most preferably this amount is 25 weight percent. However, in the case of the monomers N-alkylacrylamide, vinyl acetate, vinyl alcohol, and acrylate esters, solubility problems are created when the proportion of these anionic monomers is at the higher end of the ranges recited above for the overall ampholyte terpolymer. Thus, the N-alkylacrylamide monomers should be present in amounts of from 1 to 10 percent by weight; the vinyl acetate, vinyl alcohol, and acrylate ester monomers should be present in amounts of from 1 to 30 percent by weight as components of the overall ampholyte terpolymer.

As will be appreciated by the synthetic polymer chemist, the vinyl alcohol monomer will most conveniently be generated as a monomer simply by hydrolysis of vinyl acetate. Similarly, acrylic acid and methacrylic acid may be conveniently introduced into the terpolymer by hydrolysis of acrylamide and methacrylamide, respectively.

The next component of the ampholyte terpolymers employed in the compositions of the present invention is the cationic monomer, for example, derivatives of diallylamine, especially dimethyldiallylammonium chloride (DMDAAC). More generally, the cationic monomer may comprise 1 or 2 members independently selected from the group consisting of the following monomers and derivatives thereof:

dimethyldiallylammonium chloride (DMDAAC)
diallylamine (DAA)
methyldiallylamine (MDAA)
N,N-dialkyldiallylammonium chloride ($R_1$, $R_2$, higher than $CH_3$ in Formula 2 below)
dimethylaminoethylmethacrylate (DMAEM)
methacryloyloxyethyl trimethylammonium chloride (METAC)
methacryloyloxyethyl trimethylammonium methyl sulfate (METAMS)
acryloyloxyethyl trimethylammonium chloride (AETAC)
dimethylaminopropylmethacrylamide (DMAPMA)
methacrylamidopropyl trimethylammonium chloride (MAPTAC)
and acrylamidopropyl trimethylammonium chloride (APTAC)

Derivatives of the above cationic monomers are well known and are within the scope of the cationic monomer component (b) of the terpolymer. For example, dimethyldiallylammonium chloride, methyldiallylamine, and N,N-dialkyldiallylammonium chloride may be regarded as derivatives of diallylamine, and these together with other known derivatives of diallylamine suitable for use in the ampholyte terpolymers of the present invention may be represented by

the following general formula:

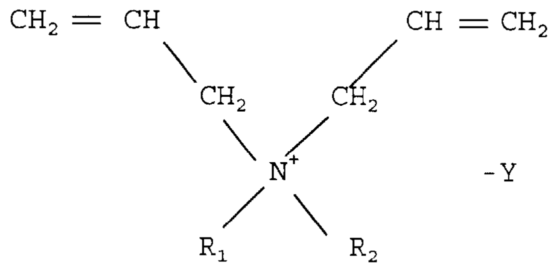

(2)

where $R_1$ and $R_2$ are independently H or $C_{1-22}$alkyl. The moiety -Y is a suitable anion, such as halide, preferably -Cl, but also including sulfate, and so forth. It is within the skill of the artisan to choose such an anion. The preferred cationic monomer of the present invention is dimethyldiallylammonium chloride (DMDAAC), ie, where $R_1$ and $R_2$ are $CH_3$.

The cationic dimethyldiallylammonium chloride monomer portion of the ampholyte terpolymers employed in compositions embodying the present invention is present in an amount of from 5 to 80 weight percent of the total terpolymer. Preferably, this amount is from 15 to 60 weight percent, more preferably 50 weight percent. However, in the case of the monomers N,N-dialkyldiallylammonium chloride, ie, where $R_1$ and $R_2$ are alkyl higher than methyl in the formula above, solubility problems are created when the proportion of these cationic monomers is at the higher end of the ranges recited above for the overall ampholyte terpolymer. Thus, the N,N-dialkyldiallylammonium chloride monomers should be present in amounts of from 1 to 10 percent by weight as components of the overall ampholyte terpolymer.

The final component of the ampholyte terpolymers employed in the compositions of the present invention is the anionic monomer, for example, acrylic acid (AA). The anionic monomer may comprise 1 or 2 members independently selected from the group consisting of the following monomers and derivatives thereof:

acrylic acid (AA)
methacrylic acid (MAA)
2-acrylamido-2-methylpropanesulfonic acid (AMPSA)
crotonic acid (CA)
sodium vinyl sulfonate (SVS)
acrylamidoglycolic acid (AGly)
2-acrylamido-2-methylbutanoic acid (AMBA)
2-acrylamido-2-methylpropanephosphonic acid (AMPPA)
sodium vinyl phosphonate (SVP)
and allyl phosphonic acid (APA)

Derivatives of the above anionic monomers are well known and are within the scope of component (c) of the terpolymer. For example, methacrylic acid may be regarded as a derivative of acrylic acid, and it, together with other known derivatives of acrylic acid suitable for use in the ampholyte terpolymers of the present invention, may be represented by the following general formula:

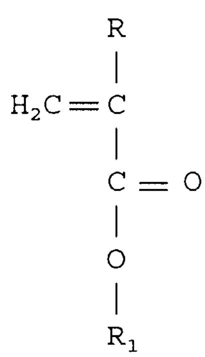

(3)

where R is H or $CH_3$; and $R_1$ is $X^+$, H, $C_{1-4}$alkyl, $CH_2CH_2OH$, $(CH_2CH_2O\text{--})_x$--H, where x=1 to 50, or phenyl, and $X^+$ is a suitable cation forming a salt of the carboxylic acid, such as sodium, potassium, ammonium, monoethanolamine, etc., all of which are well known and within the ordinary skill of the artisan to choose.

The anionic acrylic acid monomer portion of the ampholyte terpolymers employed in the compositions of the present invention is present in an amount of from 1 per cent to 75 weight percent of the total terpolymer. Preferably, this amount is from 5 per cent to 40 weight percent, more preferably 25 weight percent. However, in the case of the monomers acrylamidoglycolic acid, solubility problems are created when the proportion of these anionic monomers is at the higher end of the ranges recited above for the overall ampholyte terpolymer. Thus, the acrylamidoglycolic acid monomers should be present in amounts of from 1 per cent to 10 percent by weight as components of the overall ampholyte terpolymer.

In amounts as little as 1 per cent to 2 weight percent, although the amount present may be more, the acrylic acid measurably improves the compatibility of the overall terpolymer with anionic surfactants of which a typical cleansing product is made.

One embodiment of the present invention uses the subclass of ampholyte terpolymers wherein the cationic and anionic components are present in mole percent amounts such that the percent net charge of the overall ampholyte terpolymer is between -5.0 and +5.0. It is desirable in this embodiment that the percent net charge be as near to zero as possible. The percent net charge of the overall ampholyte terpolymer can be readily determined in accordance with the following equation:

% Net Charge = Mole % Cationic Monomer - Mole %

Anionic Monomer

Examples of specific preferred ampholyte terpolymers within the scope of the present invention are set out in the following tables:

**Specific Ampholyte Terpolymers**

Examples of specific preferred ampholyte terpolymers within the scope of the present invention are the following:

| | |
|---|---|
| AM/DMDAAC/AGly | acrylate ester/DMDAAC/AA |
| AM/DMDAAC/AMBA | AM/DMDAAC/AA/AMPSA/AMPPA |
| AM/DMDAAC/APA | acrylate ester/DMDAAC/AMBA |
| AM/DMDAAC/AMPPA | AM/DMDAAC/SVP |
| AAlc/DMDAAC/AA | acrylate ester/MAPTAC/AA |

Examples of specific more preferred ampholyte terpolymers within the scope of the present invention are the following:

| | |
|---|---|
| AM/MDAA/AA | VP/DAA/AA |
| VP/METAMS/MAA/AA | VP/DMDAAC/AMPSA |
| MAM/METAMS/MAA | AM/VP/DMDAAC/AMPSA |
| AM/VP/METAMS/MAA | AM/VP/DMDAAC/AA/AMPSA |
| AM/VP/METAMS/MAA/ AA | AM/DMDAAC/AETAC/AA |
| VP/AETAC/AA | AM/DMDAAC/DMAEM/AA |

Examples of specific most preferred ampholyte terpolymers within the scope of the present invention are the following:

| | |
|---|---|
| AM/DMDAAC/MAA | AM/MAPTAC/AA |
| AM/DMDAAC/CA | VP/DMDAAC/AA |
| AM/DMDAAC/SVS | AM/VP/DMDAAC/AA |
| AM/DAA/AA | AM/MAM/DMDAAC/AA |

(continued)

| | |
|---|---|
| AM/DAA/MAA | AM/NAAM/DMDAAC/AA |
| AM/DMAEM/AA | AM/DAA/AMPSA |
| AM/AETAC/AA | AM/VA/VOH/DMDAAC/AA |
| AM/METAMS/MAA | VP/METAMS/MAA |
| AM/DMDAAC/AA | MA/MAPTAC/AA |

The molecular weight of the ampholyte terpolymers may be within the broad range of from about 10 thousand to about 10 million, preferably about 10 thousand to about 6 million.

The ampholyte terpolymers may be prepared in a straightforward manner by using the process described immediately below.

For each terpolymer composition the appropriate weights of the aqueous nonionic component, for example acrylamide, and cationic component, for example DMDAAC, monomers are charged to a glass reactor equipped with stirring. The volume of anionic component, for example, acrylic acid, to be added is diluted first with deionized water and then added to the reactor with vigorous stirring to give a total monomer concentration of 14-20%. The monomer mixture is adjusted to pH 6.5 with dilute NaOH, heated to 55°C, and purged with nitrogen for at least thirty minutes. Polymerization is initiated by adding $5 \times 10^{-2}$ mole percent of sodium persulfate and $2.4 \times 10^{-3}$ mole percent of sodium bisulfite. After the peak exotherm is reached, additional dilution water and sodium bisulfite are added to scavenge any residual monomer and to dilute the final product to 4-8 percent polymer solids.

The ampholyte terpolymers are used in cleansing compositions by incorporating them in a cosmetically acceptable medium in amounts of from 0.05 per cent to 10 per cent by weight of said terpolymer. As used herein, the term "cosmetically acceptable medium" refers to any medium acceptable in the art for use in products that contain a surfactant system and that consumers will use in direct contact with the skin, including soap and personal hygiene products including but not limited to shower gels, liquid hand soaps, bar soaps, bath oil bars, cleansing gels and vaginal douches. Laundry and dish detergents (washing up liquids) are also within the scope of the present invention. Products intended for use in hair care, including but not limited to, shampoo, conditioner, rinsing lotion, a cream or a treatment product applied before or after coloring or blending, before or after perming or before or after straightening a colouring product, a setting lotion, a brushing lotion, a bleaching product, a perming product, or a straightening product, are not within the scope of the compositions of the present invention.

The cosmetically acceptable medium may contain ingredients other than the ampholyte terpolymer which are known in the art for the formulation of the type of product to be made, eg shower gel, bar soap, etc.

The cosmetically acceptable media compositions of the present invention may contain one or more surfactants selected from all manner of anionic, nonionic, zwitterionic, amphoteric or cationic surfactants. Typically, the surfactant will be present at from about 1 percent to about 70 percent, preferably about 3 percent to 35 percent by weight.

Most preferably, the cleansing compositions of the present invention contain anionic surfactants which, for example, include alkylether sulfates, olefin sulfonates, alkyl and alkenyl sulfates, alkyl sulfonates, alkylbenzene sulfonates, ammonium lauryl sulfate and ammonium lauryl ether sulfate. An unexpected discovery associated with the present invention is that the amphoteric terpolymer, when used with a surface active agent, results in reduced dermal and ocular irritation when compared with similar surfactant-containing compositions that do not contain the ampholyte terpolymer.

Soap bars embodying the present invention may either contain real soap, combinations of soap and natural or synthetic surfactants, or may be formulated solely with natural or synthetic surfactants, such as alkylbenzene sulfonates.

According to the present invention in a second aspect a method for cleaning human skin other than head hair cleansing comprising the steps of applying to the skin a cleansing composition according to the first aspect of the present invention and washing the skin with water and the composition.

The products, and methods of using the same, of the present invention provide an advance over the art in that the user of the products and methods will experience reduced dermal and/or ocular irritation when compared with use of similar products that do not contain the ampholyte terpolymers described herein.

As stated above, the ampholyte terpolymers employed in the compositions of the present invention are effective in reducing dermal and/or ocular irritation when used in an amount ranging between about 0.05 per cent and 10 weight per cent of the total cleaning product formulation. Use of the ampholyte terpolymers in the concentrations disclosed will also result in cleaning products having the desired qualities of suitable lather production, good skin feel and lubricity.

An ampholyte terpolymer containing 25 weight percent AM, 50 weight percent DMDAAC and 25 weight percent AA is commercially available from Calgon Corporation, Pittsburgh, PA as MERQUAT* Plus 3330 (*Trade Mark). An

ampholyte terpolymer containing 38 weight percent AM, 45 weight percent DMDAAC and 17 weight percent AA is commercially available from Calgon Corporation as MERQUAT* Plus 3331. Also available from Calgon Corp. are ampholyte terpolymers containing MA, MAPTAC and acrylic acid in ratios of 25:50:25 (MERQUAT* 2001) and 45:45:10 (MERQUAT* 2001N).

## EXAMPLES

The following Examples are intended to illustrate the invention by way of example only.

### Example 1

Various shower gel formulations were prepared as follows:

Table 1

| Ingredient | Sample %w/w | | | |
|---|---|---|---|---|
| | A | B | C | D |
| 1. Water | 33.3 | -- | 22.2 | 27.7 |
| 2. Sodium Cocoamphoacetate | 14.0 | 14.0 | 14.0 | 14.0 |
| 3. Disodium lauryl sulfosuccinate | 30.0 | 30.0 | 30.0 | 30.0 |
| 4. Cocamidopropyl betaine | 10.0 | 10.0 | 10.0 | 10.0 |
| 5. Isostearamideopropyl morpholine lactate | 6.0 | 6.0 | 6.0 | 6.0 |
| 6. Ethylene glycol distearate | 3.5 | 3.5 | 3.5 | 3.5 |
| 7. Sodium chloride | 3.0 | 3.0 | 3.0 | 3.0 |
| 8. Merquat® Plus 3330 | -- | 33.3 | 11.1 | 5.6 |
| 9. DMDM Hydantoin | 0.2 | 0.2 | 0.2 | 0.2 |

Sample B contained 3% solids of Merquat® Plus 3330, Sample C 1% solids and Sample D 0.5% solids.

Sample A, which was a control, was prepared by mixing the first six ingredients listed in Table 1 and heating to about 70°C. The ingredients were blended slowly, and the pH of the mixture was adjusted with citric acid to 6.3 ±0.1. The mixture was removed from the heat, and the sodium chloride was mixed in. When the product was homogenous, the hydantoin was added and mixed. The mixture was cooled to about 50°C. Water was added, as necessary to make up for heating loss. The pH was checked at 22°C.

Samples B through D were prepared by mixing ingredients 1, 2, 3, 4, 5, 6 and 8 and heating the mixture to 70°C. The ingredients were slowly blended, and adjusted to a pH of 6.3 ±0.1 with citric acid. The sodium chloride was added and mixed in. When the product was homogenous, the hydantoin was added with mixing. The mixture was cooled to about 50°C, and water, if any, was added. The pH was checked at 26°C.

### Example 2

Samples A to D were sent to Tox Monitor Laboratories, Inc, Oak Park, Illinois for dermal irritation analysis.

New Zealand white rabbits approximately 8 to 10 weeks old were used for this study. The rabbits were obtained from Kuiper Rabbitry, Gary, Indiana. The rabbits were individually identified by ear tags. The rabbits were individually housed in stainless steel cages in a temperature, humidity, and light controlled room. The rabbits were maintained according to the recommendations contained in the DAGW publication No 86-23 (NIH) : "Guide for the Care and Use cf Laboratory Animals". They were conditioned for at least 4 days prior to study initiation. Purina Rabbit Chow and water were available *ad libitum.* All animals used for this study were considered to be in good health at study initiation.

The day before study initiation, electric clippers were used to remove the hair from the abdomen and the sides of 6 rabbits. Two sites, one unabraded skin and one abraded skin, approximately 1 square inch each were designated for application of test material. A 0.5 ml aliquot of the test material was moistened and applied to each site, covered with a two-layer gauze patch secured with non-irritating porous adhesive tape and the entire site was covered with a 4 mil plastic wrap and secured with more adhesive tape. At the end of the 4 hour contact period, excess material was removed from the sites.

The study was terminated at the end of 120 hours.

Dermal irritation observations were performed at 24 hours after the test material was applied to the sites and again at 72 hours following the final exposure (120 hours). Grading and scoring of irritation were performed in accordance with the Draize scoring system. Primary dermal irritation score calculations are presented in Tables 2 through 5, for Samples A through D, respectively.

**Table 2**

**Sample A Results**

**Primary dermal irritation**

| | 24 HOURS | | | | 48 HOURS | | | |
|---|---|---|---|---|---|---|---|---|
| | UNABRADED | | ABRADED | | UNABRADED | | ABRADED | |
| Rabbit Number | ER | ED | ER | ED | ER | ED | ER | ED |
| 380 | 0 | 0 | 1 | 0 | 1 | 0 | 1 | 0 |
| 381 | 0 | 0 | 0 | 0 | 1 | 1 | 1 | 1 |
| 382 | 0 | 0 | 1 | 0 | 2 | 1 | 2 | 1 |
| 383 | 0 | 0 | 0 | 0 | 1 | 1 | 1 | 1 |
| 384 | 0 | 0 | 0 | 0 | 1 | 1 | 1 | 1 |
| 385 | 1 | 0 | 1 | 0 | 2 | 1 | 2 | 1 |
| Average | 0.17 | 0 | 0.5 | 0 | 1.33 | 0.83 | 1.33 | 0.83 |

| | 72 HOURS | | | | 120 HOURS | | | |
|---|---|---|---|---|---|---|---|---|
| | UNABRADED | | ABRADED | | UNABRADED | | ABRADED | |
| Rabbit Number | ER | ED | ER | ED | ER | ED | ER | ED |
| 380 | 2 | 1 | 2 | 1 | 2 | 1 | 2 | 1 |
| 381 | 2 | 2 | 2 | 2 | 2 | 2 | 2 | 2 |
| 382 | 3 | 2 | 3 | 2 | 3 | 1 | 3 | 1* |
| 383 | 2 | 2 | 2 | 2 | 2 | 2 | 2 | 2 |
| 384 | 2 | 2 | 2 | 2 | 3 | 1 | 3 | 1* |
| 385 | 3 | 3 | 3 | 3 | 3 | 2 | 3 | 2* |
| Average | 2.33 | 2.0 | 2.33 | 2.0 | 2.5 | 1.5 | 2.5 | 1.5 |

24 Hours = 0.34

48 Hours = 2.16

72 Hours = 4.33

120 Hours = 4.00

Primary skin irritation index (72 and 120 hour score average) 4.17

ER = Erythema

ED = Edema

* blanching and slight fissuring

**Table 3**

**Sample B Results**

**Primary dermal irritation**

| | 24 HOURS | | | | 48 HOURS | | | |
|---|---|---|---|---|---|---|---|---|
| | UNABRADED | | ABRADED | | UNABRADED | | ABRADED | |
| Rabbit Number | ER | ED | ER | ED | ER | ED | ER | ED |
| 386 | 0 | 0 | 0 | 0 | 1 | 0 | 1 | 0 |
| 387 | 0 | 0 | 1 | 0 | 0 | 0 | 1 | 0 |
| 388 | 1 | 0 | 1 | 0 | 0 | 0 | 1 | 0 |
| 389 | 0 | 0 | 1 | 0 | 0 | 0 | 1 | 0 |
| 390 | 1 | 0 | 1 | 0 | 1 | 1 | 2 | 1 |
| 391 | 0 | 0 | 0 | 0 | 1 | 0 | 1 | 0 |
| Average | 0.33 | 0 | 0.67 | 0 | 0.5 | 0.17 | 1.17 | 0.17 |

| | 72 HOURS | | | | 120 HOURS | | | |
|---|---|---|---|---|---|---|---|---|
| | UNABRADED | | ABRADED | | UNABRADED | | ABRADED | |
| Rabbit Number | ER | ED | ER | ED | ER | ED | ER | ED |
| 386 | 1 | 0 | 1 | 0 | 1 | 0 | 1 | 0 |
| 387 | 1 | 0 | 1 | 0 | 2 | 0 | 2 | 0 |
| 388 | 1 | 1 | 1 | 1 | 2* | 0 | 2 | 0 |
| 389 | 1 | 1 | 2 | 1 | 1 | 0 | 2 | 0 |
| 390 | 2 | 2 | 2 | 2 | 2* | 1 | 2 | 1 |
| 391 | 2 | 1 | 2 | 1 | 2* | 1 | 2 | 1 |
| Average | 1.33 | 0.83 | 1.5 | 0.83 | 1.67 | 0.33 | 1.83 | 0.33 |

24 Hours = 0.5

48 Hours = 1.00

72 Hours = 2.25

120 Hours = 2.08

Primary skin irritation index (72 and 120 hour score average) 2.16

ER = Erythema

ED = Edema

* slight flaking of epidermis

**Table 4**

**Sample C Results**

**Primary dermal irritation**

| | 24 HOURS | | | | 48 HOURS | | | |
|---|---|---|---|---|---|---|---|---|
| | UNABRADED | | ABRADED | | UNABRADED | | ABRADED | |
| Rabbit Number | ER | ED | ER | ED | ER | ED | ER | ED |
| 475 | 1 | 0 | 1 | 0 | 1 | 0 | 2 | 0 |
| 476 | 0 | 0 | 1 | 0 | 1 | 0 | 1 | 0 |
| 477 | 1 | 0 | 1 | 0 | 1 | 0 | 1 | 0 |
| 478 | 1 | 0 | 1 | 0 | 1 | 0 | 1 | 0 |
| 479 | 1 | 0 | 1 | 0 | 1 | 0 | 1 | 0 |
| 480 | 0 | 0 | 1 | 0 | 1 | 1 | 1 | 1 |
| Average | 0.67 | 0 | 1.0 | 0 | 1.0 | 0.17 | 1.17 | 0.17 |

| | 72 HOURS | | | | 120 HOURS | | | |
|---|---|---|---|---|---|---|---|---|
| | UNABRADED | | ABRADED | | UNABRADED | | ABRADED | |
| Rabbit Number | ER | ED | ER | ED | ER | ED | ER | ED |
| 475 | 2 | 1 | 2 | 1 | 1 | 0 | 2 | 0 |
| 476 | 2 | 1 | 2 | 1 | 2 | 0 | 2 | 0 |
| 477 | 1 | 1 | 1 | 1 | 1 | 0 | 1 | 0 |
| 478 | 2 | 2 | 2 | 2 | 2 | 2 | 3 | 2* |
| 479 | 2 | 2 | 2 | 2 | 2 | 1 | 2 | 1 |
| 480 | 1 | 1 | 1 | 1 | 1 | 0 | 2 | 0 |
| Average | 1.67 | 1.33 | 1.67 | 1.33 | 1.5 | 0.5 | 2.0 | 0.5 |

24 Hours = 0.84

48 Hours = 1.26

72 Hours = 3.00

120 Hours = 1.80

Primary skin irritation index (72 and 120 hour score average) 2.63

ER = Erythema

ED = Edema

* fissuring

Coriaceousness and scaling on all animals.

**Table 5**

**Sample D Results**

**Primary dermal irritation**

| | 24 HOURS | | | | 48 HOURS | | | |
|---|---|---|---|---|---|---|---|---|
| | UNABRADED | | ABRADED | | UNABRADED | | ABRADED | |
| Rabbit Number | ER | ED | ER | ED | ER | ED | ER | ED |
| 530 | 0 | 0 | 1 | 0 | 1 | 1 | 1 | 1 |
| 531 | 0 | 0 | 1 | 0 | 1 | 0 | 1 | 0 |
| 532 | 0 | 0 | 1 | 0 | 1 | 0 | 1 | 0 |
| 533 | 0 | 0 | 1 | 0 | 1 | 0 | 1 | 0 |
| 534 | 0 | 0 | 1 | 0 | 0 | 0 | 1 | 0 |
| 535 | 1 | 0 | 1 | 1 | 1 | 1 | 2 | 1 |
| Average | 0.17 | 0 | 1.0 | 0.17 | 0.83 | 0.33 | 1.17 | 0.33 |

| | 72 HOURS | | | | 120 HOURS | | | |
|---|---|---|---|---|---|---|---|---|
| | UNABRADED | | ABRADED | | UNABRADED | | ABRADED | |
| Rabbit Number | ER | ED | ER | ED | ER | ED | ER | ED |
| 530 | 2 | 3 | 2 | 3 | 3 | 2 | 3 | 2 |
| 531 | 2 | 2 | 2 | 2 | 3 | 1 | 3 | 2 |
| 532 | 1 | 2 | 1 | 2 | 2 | 1 | 2 | 2 |
| 533 | 2 | 2 | 2 | 2 | 3 | 2 | 3 | 2 |
| 534 | 1 | 2 | 1 | 2 | 2 | 1 | 2 | 1 |
| 535 | 2 | 4 | 3 | 4 | 3 | 2 | 3 | 2 |
| Average | 1.67 | 2.5 | 1.83 | 2.5 | 2.67 | 1.5 | 2.67 | 1.5 |

24 Hours = 0.67

48 Hours = 1.33

72 Hours = 4.25

120 Hours = 4.17

Primary skin irritation index (72 and 120 hour score average) 4.21

ER = Erythema

ED = Edema

**EVALUATION OF DERMAL REACTIONS**

| | | |
|---|---|---|
| A. | Erythema and Eschar Formation: | |
| | Very slight erythema (barely perceptible) | 1 |
| | Well-defined erythema | 2 |
| | Moderate to severe erythema | 3 |
| | Severe erythema (beet redness) to slight eschar formation (injuries in depth) | 4 |
| B. | Edema Formation: | |
| | Very slight edema (barely perceptible) | 1 |
| | Slight edema (edges of area well defined by definite raising) | 2 |
| | Moderate edema (area raised approx. 1 mm) | 3 |
| | Severe edema (raised more than 1 mm and extending beyond area of exposure) | 4 |
| * The value recorded for each reading is the average value of the animals subject to the test. | | |

**PRIMARY DERMAL IRRITATION DESCRIPTIVE RATING**

| Mean Primary Irritation Score (values) | Descriptive Rating |
|---|---|
| 0 | Non-Irritating |
| 0.1-0.5 | Minimally Irritating |
| 0.6-1.5 | Slightly Irritating |
| 1.6-3.0 | Mildly Irritating |
| 3.1-5.0 | Moderately Irritating |
| 5.1-6.5 | Severely Irritating |
| 6.6-8.0 | Extremely Irritating |

As can be seen from the results presented above, the primary skin irritation index, which was the average of the 72 and 120 hour scores, was 4.17 for the control sample, containing no ampholyte terpolymer. The primary skin irritation index was substantially reduced to 2.16 and 2.63 for Samples B and C, respectively. These samples contained 3 percent and 1 percent ampholyte terpolymer solids, respectively. The sample containing only 0.5 percent solids of the ampholyte terpolymer, Sample D, had a primary skin irritation index of 4.21, which was comparable to the response seen with the control sample. Thus, samples containing at least 1 percent ampholyte terpolymer solids were effective in reducing dermal irritation.

**Example 3 - Liquid Hand Soap**

The following is a formulation representing a liquid hand soap composition embodying the present invention.

| Weight Percent | Ingredient |
|---|---|
| 65.09 | Water |
| 22.0 | Sodium C14-16 Olefin Sulfonate |
| 3.0 | Cocamidopropyl Betaine |
| 3.0 | Lauramide DEA |
| 0.53 | Chloroxylenol |
| 0.13 | Tetrasodium EDTA |
| 2.00 | Sodium Chloride |
| 0.25 | Citric Acid |
| 2.00 | MERQUAT® 2001 |
| 2.00 | MERQUAT® Plus 3330 |

The amphoteric terpolymer in this product will provide reduced dermal and ocular irritation than a similar product not containing the terpolymer, while at the same time providing a product that results in suitable lather. Similar properties will be obtained using amphoteric terpolymer concentrations of up to about 10.0 percent with proportionate reductions in other ingredients.

**Example 4 - Bath Oil Bar**

The following is a formulation representing a bath oil bar composition embodying the present invention.

| Weight Percent | Ingredient |
|---|---|
| 2.00 | Water |
| 10.00 | Propylene Glycol |
| 0.50 | MERQUAT® Plus 3331 |
| 0.50 | MERQUAT® 2001 |
| 69.00 | PPG-3 Myristyl Ether |
| 10.00 | Mineral Oil |
| 8.00 | Sodium Stearate |

The amphoteric terpolymer in this product will result in reduced dermal and ocular irritation than a similar product not containing the terpolymer. Similar results will be obtained using up to about 10 percent amphoteric terpolymer with proportionate reductions in other ingredients.

**Example 5 - Fine Fabric Detergent**

The following is a formulation representing a fine fabric detergent composition according to the present invention.

| Weight Percent | Ingredient |
|---|---|
| 5.0 | Cocamine Oxide |
| 2.0 | Cocamide DEA |
| 15.0 | Sodium Linear Alkylate Sulfonate (60% solution) |

(continued)

| Weight Percent | Ingredient |
|---|---|
| 25.0 | Sodium Alpha Olefin Sulfonate (40% solution) |
| 0.25 | Citric Acid, Anhydrous |
| 4.0 | MERQUAT® 2001N |
| 48.75 | Water |

The amphoteric terpolymer in this product will result in reduced dermal and ocular irritation than a similar product not containing the terpolymer. Similar results will be obtained using up to about 10 percent amphoteric terpolymer with proportionate reductions in other ingredients.

**Example 6 - Light Duty Dishwashing Detergent**

The following is a formulation representing a light duty dishwashing detergent composition according to the present invention.

| Weight Percent | Ingredient |
|---|---|
| 5.0 | Sodium Xylene Sulfonate |
| 3.0 | Standamid SD |
| 15.0 | Lauryl Polyglucose |
| 30.0 | Sodium AOS (40%) |
| 2.0 | MERQUAT® Plus 3330 |
| 1.5 | MERQUAT® 2001N |
| 43.5 | Water |

The amphoteric terpolymers in this product will result in reduced dermal and ocular irritation than a similar product not containing the terpolymer. Similar results will be obtained using up to about 10 percent amphoteric terpolymer with proportionate reductions in other ingredients.

**Example 7 - Baby Bath**

| Weight Percent | Ingredient |
|---|---|
| 2.0 | MERQUAT® 2001 |
| 22.0 | Sodium Laureth Sulfate |
| 20.0 | Sodium Laureth-11 Carboxylate |
| 12.0 | Cocoamidopropyl Betaine |
| 2.0 | Cocamide DEA |
| q.s. to 100 | Water |
| 2.0 | Merquat® Plus 3331 |
| 0.5 | Camomile Special (Dragoco) |
| 1.0 | Avocado Special (Dragoco) |
| q.s. | Perfume |
| q.s. | Preservative |
| q.s. | Magnesium Sulfate x7 $H_2O$ |

The sodium laureth sulfate and sodium laureth-11 carboxylate can be obtained from Hüls as Marlinat® 242/28 and Marlinat® CM 105, respectively. The cocamidopropyl Betaine can be obtained from Hüls as Ampholyt JB 130 and the cocamide DEA can also be obtained from Hüls as Marlamide® M 1218.
The amphoteric terpolymer in this product will result in reduced dermal irritation than a similar product not containing the terpolymer. Similar results will be obtained using up to about 10 percent amphoteric terpolymer with proportionate reductions in other ingredients.

**Example 8 - All-Purpose Cleaner**

| Weight Percent | Ingredient |
|---|---|
| 79.0 | Deionized Water |
| 3.0 | Tetrapotassium Pyrophosphate |
| 1.0 | Sodium Gluconate |
| 1.0 | Ethanolamine (MEA) |
| 2.0 | Ethoxylated Octyl Phenol |
| 3.0 | Modified Coconut Diethanolamine |
| 8.0 | Glycol Ether |
| 3.0 | MERQUAT® Plus 3331 |

Tetrapotassium pyrophosphate can be obtained from FMC Corp. Ethoxylated octyl phenol can be obtained from Union Carbide as Triton® x-102. Coconut diethanolamine can be obtained from Stepan Chemical Co., Northfield, Illinois as Ninol® 11 CM.
The amphoteric terpolymer in this product will result in reduced dermal irritation than a similar product not containing the terpolymer. Similar results will be obtained using up to about 10 percent amphoteric terpolymer with proportionate reductions in other ingredients.

**Example 9 - Laundry Detergent**

| Weight Percent | Ingredient |
|---|---|
| 29.0 | Water |
| 5.0 | Sodium Citrate |
| 5.0 | Sodium Xylene Sulfonate |
| 15.0 | Lauryl Polyglucose |
| 5.0 | Ethanol |
| 38.0 | Sodium Lauryl Ether Sulfate |
| 2.0 | MERQUAT® Plus 3330 |
| 1.0 | MERQUAT® 2001 |

The sodium xylene sulfonate used here and in Example 6 can be obtained as Sulfotex SXS 40 from Henkel. Sodium lauryl ether sulfate can also be obtained from Henkel as Sulfotex 6040S. Lauryl polyglucose can be obtained from Henkel as Glucopan 600 CSUP.
The amphoteric terpolymers in this product will result in reduced dermal irritation than a similar product not containing the terpolymer. Similar results will be obtained using up to about 10 percent amphoteric terpolymer with proportionate reductions in other ingredients.
Whereas particular embodiments of this invention have been described above for purposes of illustration, it will be evident to those skilled in the art that numerous variations of the details of the present invention may be made

without departing from the invention as defined in the appended claims.

**Claims**

1. A cleansing composition comprising a cosmetically acceptable medium containing from 0.05 per cent by weight to 10 per cent by weight of one or more ampholyte terpolymers having a weight average molecular weight of from 10,000 to 10,000,000 comprising:

   (a) a non-ionic monomer forming from 1 per cent to 95 per cent by weight of the terpolymer;
   (b) a cationic monomer forming from 5 per cent to 80 per cent by weight of the terpolymer; and
   (c) an anionic monomer forming from 1 per cent to 75 per cent of the terpolymer;

   and wherein the cosmetically acceptable medium comprises a medium suitable for providing a hydrophilic cleansing product for use in direct contact with the skin of a user but not primarily for hair care, the medium containing a surfactant.

2. A composition according to Claim 1 and wherein the said medium contains from 3 per cent to 35 per cent by weight of one or more surfactants.

3. A composition according to claim 2 and wherein the said one or more surfactants comprise one or more anionic surfactants.

4. A composition according to any one of claims 1 to 3 and wherein the composition comprises or is incorporated within a soap or personal hygiene product.

5. A composition according to claim 4 and wherein the composition comprises or is incorporated within a product comprising a shower gel, liquid hand soap, bar soap, bath oil bar, cleansing gel or body douche.

6. A composition according to any one of claims 1 to 3 and wherein the composition comprises or is incorporated in a laundry detergent or a washing up liquid.

7. A composition according to any one of the preceding claims and wherein the terpolymer has an overall per cent net charge of from -5.0 to +5.0.

8. A composition according to any one of claims 1 to 5 and wherein the terpolymer comprises:

   (a) from at least 1 per cent to as much as 95 weight per cent of the nonionic monomer comprising from 1 to 3 members independently selected from the group consisting of the following monomers and derivatives thereof:

| | |
|---|---|
| acrylamide (AM) | vinylacetate(VA) |
| N-alkylacrylamide (NAAM) | vinyl alcohol (VOH) |
| N-vinylpyrrolidinone (VP) | acrylate esters |
| methacrylamide (MAM) | allyl alcohol (AAlc) |
| methyl acrylate (MA) | methyl methacrylate (MMA) |
| and methacrylate esters | |

   (b) from at least 5 per cent to as much as 80 weight per cent of a cationic monomer comprising 1 or 2 members independently selected from the group consisting of the following monomers and derivatives thereof:

      dimethyldiallylammonium chloride (DMDAAC)
      diallylamine (DAA)
      methyldiallylamine (MDAA)
      N,N-dialkyldiallylammonium chloride
      dimethylaminoethylmethacrylate (DMAEM)
      methacryloyloxyethyl trimethylammonium chloride (METAC)

methacryloyloxyethyl trimethylammonium methyl sulfate (METAMS)
acryloyloxyethyl trimethylammonium chloride (AETAC)
dimethylaminopropylmethacrylamide (DMAPMA)
methacrylamidopropyl trimethylammonium chloride (MAPTAC)
and acrylamidopropyl trimethylammonium chloride (APTAC)

(c) from at least 1 per cent to as much as 75 weight per cent of an anionic monomer comprising 1 or 2 members independently selected from the group consisting of the following monomers and derivatives thereof:

acrylic acid (AA)
methacrylic acid (MAA)
2-acrylamido-2-methylpropanesulfonic acid (AMPSA)
crotonic acid (CA)
sodium vinyl sulfonate (SVS)
acrylamidoglycolic acid (AGly)
2-acrylamido-2-methylbutanoic acid (AMBA)
2-acrylamido-2-methylpropanephosphonic acid (AMPPA)
sodium vinyl phosphonate (SVP)
and allyl phosphonic acid (APA).

**9.** A composition according to any one of the preceding claims and wherein the weight percent of the nonionic component is from 10 to 80, the weight percent of the cationic component is from 15 to 60, and the weight percent of the anionic component is from 5 to 40.

**10.** A composition according to any one of the preceding claims and wherein the ampholyte terpolymer is selected from the group consisting of

| | |
|---|---|
| AM/DMDAAC/AGly | acrylate ester/DMDAAC/AA |
| AM/DMDAAC/AMBA | AM/DMDAAC/AA/AMPSA/AMPPA |
| AM/DMDAAC/APA | acrylate ester/DMDAAC/AMBA |
| AM/DMDAAC/AMPPA | AM/DMDAAC/SVP |
| AAlc/DMDAAC/AA | AM/DMDAAC/AA |
| AM/MDAA/AA | VP/DAA/AA |
| VP/METAMS/MAA/AA | VP/DMDAAC/AMPSA |
| MAM/METAMS/MAA | AM/VP/DMDAAC/AMPSA |
| AM/VP/METAMS/MAA | AM/VP/DMDAAC/AA/AMPSA |
| AM/VP/METAMS/MAA/ AA | AM/DMDAAC/AETAC/AA |
| | AM/MAPTAC/methacrylate |
| VP/AETAC/AA | AM/DMDAAC/DMAEM/AA |
| AM/DMDAAC/MAA | MA/MAPTAC/AA |
| AM/DMDAAC/CA | VP/DMDAAC/AA |
| AM/DMDAAC/SVS | AM/VP/DMDAAC/AA |
| AM/DAA/AA | AM/MAM/DMDAAC/AA |
| AM/DAA/MAA | AM/NAAM/DMDAAC/AA |
| AM/DMAEM/AA | AM/DAA/AMPSA |
| AM/AETAC/AA | AM/VA/VOH/DMDAAC/AA |
| AM/METAMS/MAA | VP/METAMS/MAA. |
| and acrylate ester/MAPTAC/AA | |

**11.** A composition according to any one of the preceding claims and wherein the ampholyte terpolymer has a molecular weight of from about 10 thousand to about 6 million.

**12.** A composition according to any one of Claims 1 to 7 and wherein the components of the said terpolymer are such that (a) the nonionic monomer is acrylamide, (b) the cationic monomer is dimethyldiallylammonium chloride, and (c) the anionic monomer is acrylic acid, and the weight ratio of a:b:c is in the range from 25:50:25 to 38:45:17.

13. A composition according to any one of claims 1 to 7 and wherein the components of the terpolymer are such that (a) the nonionic monomer is methyl acrylate, (b) the cationic monomer is methacrylamidopropyl trimethylammonium chloride, and (c) the anionic monomer is acrylic acid, and the weight ratio of a:b:c is in the range of from 25:50:25 to 45:45:10.

14. A method for cleaning human skin other than head hair comprising applying to said skin a composition according to any one of claims 1 to 13 and washing the skin with water and the composition.

European Patent Office

# EUROPEAN SEARCH REPORT

Application Number
EP 98 30 5161

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | EP 0 522 756 A (CALGON) 13 January 1993<br>* claims 1-10 * | 1-12,14 | A61K7/00 |
| D,X | US 5 338 541 A (G.F. MATZ ET AL) 16 August 1994<br>* column 2, line 21 - column 3, line 62; claims 1-8 * | 1 | |
| D,X | EP 0 522 755 A (CALGON) 13 January 1993<br>* claims 1-13 * | 1,7 | |
| E | WO 98 44012 A (CALGON) 8 October 1998<br>* the whole document * | 1-11,13, 14 | |

TECHNICAL FIELDS SEARCHED (Int.Cl.6)
A61K

The present search report has been drawn up for all claims

| Place of search | Date of completion of the search | Examiner |
|---|---|---|
| THE HAGUE | 24 November 1998 | WILLEKENS, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)